# EUROPEAN PATENT APPLICATION

(11) **EP 4 438 072 A1**
(43) Date of publication of application: **02.10.2024**
(21) Application number: 24167222.9
(22) Date of filing: 28.03.2024
(51) Int. Cl.: A61M 1/34, A61M 1/36

(54) **SYSTEMS AND METHODS FOR CONTROLLING THE RISK OF CITRATE REACTIONS DURING APHERESIS**

(30) Priority: 28.03.2023 US 202363492695 P; 27.03.2024 US 202418618801
(71) Applicant: Fenwal, Inc., Lake Zurich, IL 60047 (US)
(72) Inventor: GNIADEK, Thomas, Lake Zurich, 60047 (US)
(74) Representative: Maikowski & Ninnemann Patentanwälte Partnerschaft mbB

(57) **Abstract**

Methods and systems for performing an apheresis procedure with a reduced risk of citrate reaction occurrences are described. The volume of body water in the apheresis subject is used to predict the likelihood of a citrate reaction during infusion of a blood component to the subject and allows for establishing and maintaining an optimal flow rate of the infused blood component.

## Description

### Field of the Disclosure

**The** present disclosure is directed to the processing and collection of blood and its components in an apheresis system. More particularly, the present disclosure is directed to apheresis systems and methods wherein the risk of a citrate reaction in the apheresis donor or patient can be predicted and potentially mitigated. Even more particularly, the present disclosure is directed to apheresis systems and methods wherein the risk of a citrate reaction in the donor or patient can be predicted (and mitigated) based on certain donor/patient characteristics such as the amount of body water.

### Background

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. The above-described separation, collection and return process is often referred to as "apheresis." Where the goal of the apheresis procedure is platelet collection, the process may be referred to as "plateletpheresis" while a process where the goal is the collection of plasma is referred to as "plasmapheresis."

Removing only particular constituents is particularly advantageous when the blood source is a human donor, because potentially less time is needed for the donor's body to return to pre-donation levels, and donations can be made at more frequent intervals than when whole blood is collected. In addition, removing only particular constituents by apheresis may allow collection of a volume or amount of those constituents which would not be possible to safely achieve if whole blood were removed from the donor to achieve the same constituent collection yield. For example, apheresis collection of platelets, plasma, and RBCs can yield collection amounts for those constituents that could pose a risk to donor health if the same amount were to be collected by whole blood donation. Given a limited number of donors and donations, this increases the overall supply of blood constituents, such as plasma and platelets, made available for transfusion and/or therapeutic treatment.

Whole blood may be separated into its constituents through any of a number of automated procedures, such as centrifugation, membrane separation, and devices using other separation techniques. Centrifugation, for example, requires that the whole blood be passed through a centrifuge after it is withdrawn from, and before it is returned to, the blood source. To reduce contamination and possible infection (if the blood source is a human donor or patient), the blood is preferably processed within a sealed, sterile fluid flow system during the centrifugation process. Typical blood processing systems include a disposable, sealed, and sterile fluid circuit, including a centrifuge chamber portion, that is mounted in cooperation on a durable, reusable assembly containing the hardware (centrifuge, drive system, pumps, valve actuators, programmable controller, and the like) that rotates a centrifuge chamber and controls the flow through the fluid circuit.

**The** disposable fluid circuit is typically configured to allow attachment of additional components, such as bags containing fluids used during the procedure. These fluids may be used for different purposes, such as anticoagulant fluid for preventing the coagulation of blood outside of the body, storage fluid to be added to separated blood components for long-term storage, and replacement fluid to be infused into a patient or donor to replace blood components that are being stored for later use.

Sodium citrate, such as acid-citrate-dextrose or ACD, or other citrate-containing solutions are commonly used as an anticoagulant and are combined with the whole blood during withdrawal of the whole blood from the donor or patient ("the apheresis subject"). During separation of the whole blood into its components, some of the citrate added to the whole blood during anticoagulation remains with the separated component. This includes the component that is returned to the apheresis subject. Thus, the apheresis subject receives one or more blood components with some amount of citrate.

Citrate infusion into an apheresis subject may be potentially harmful by increasing the citrate concentration of blood and body fluids. Citrate binds ions such as calcium and magnesium within the body, lowering the free concentration of those ions. In particular, the lowering of free calcium levels is thought to result in the primary side effects related to citrate infusion. Importantly, citrate is also cleared from body fluids by metabolism and excretion in urine. Therefore, risks associated with citrate infusion can be considered relative to the rate of citrate infusion, the rate of citrate clearance, and the biologic susceptibility of an individual to the effects of increased citrate concentration. Symptoms of citrate toxicity may include numbness or tingling of lips or fingers, feelings of vibrations, metallic taste, chills, shivering, light-headedness, feeling of tightness, muscle twitching, rapid or slow pulse and/or shortness of breath. More severe symptoms may include muscle spasms, vomiting and, if left untreated, cardiac arrhythmia, including cardiac arrest, and/or seizure.

To mitigate and/or guard against citrate reactions, one common approach is to reduce or otherwise control the flow rate of the blood component being returned or (re)infused to the apheresis subject. However, this may result in longer than desired apheresis procedure durations and thereby reducing the number of apheresis procedures that can be scheduled per day. In the case of therapeutic apheresis, this may also result in delay of therapy, which may itself cause patient harm.

Currently, the risk of citrate reactions occurring is understood to be related to the rate of citrate infusion relative to the body weight (e.g., mg/kg/min). However, body weight may not be the ideal predictor of a citrate reaction risk.

Body water represents the fluid component in which citrate distributes in vivo. Particularly in the case of therapeutic apheresis, sometimes involving critically ill patients who may be volume overloaded at the time of an apheresis procedure, body water (e.g., citrate volume of distribution) may be markedly different from a euvolemic individual of the same weight. Furthermore, body water may be calculated more accurately in all apheresis subjects by incorporating input parameters such as height as well as whether an individual is male or female.

Thus, it would be desirable to provide a method and system where, based on certain other physical characteristics of the apheresis subject, the risk of a citrate reaction can be more reliably predicted and, for certain subjects, the rate of blood component infusion can be safely increased, thereby shortening the time of the overall procedure.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a method for controlling a risk of a citrate reaction in an apheresis subject is provided. In particular, a method for programming a controller to implement a biological fluid processing procedure for controlling a risk of a citrate reaction in an apheresis subject is provided. The method includes determining a physical parameter of an apheresis subject and determining an amount of body water in the apheresis subject based on the physical parameter. The method includes infusing a separated, citrate-anticoagulated blood component to said subject. The rate of infusion of the separated citrate-anticoagulated blood component to said subject is based at least, in part, on said amount of body water in said apheresis subject.

In another aspect, a method for establishing a citrate-anticoagulated blood component infusion rate for an apheresis procedure is provided, wherein a physical parameter of a subject selected for said apharesis procedure is determined and an amount of body water in the apheresis subject based on the physical parameter is determined. The method includes providing a separated, citrate-anticoagulated blood component for said subject and establishing an infusion rate of the separated citrate-anticoagulated blood component based at least, in part, on said amount of body water in said apheresis subject.

In another aspect, a system for carrying out an apheresis procedure is provided. The system includes a durable, reusable hardware device including a cell separator drive unit, one or more pumps and a controller. The system also includes a disposable fluid circuit configured for mounting onto said hardware device. The fluid circuit includes a separation chamber, a venous access device for establishing flow communication between an apheresis subject and the separation chamber. The circuit further includes a fluid line defining a flow path for introducing blood from the apheresis subject to the separation chamber and a fluid line for infusing a separated blood component from the chamber to the apheresis subject. The controller is configured to (a) determine the body water volume of an apheresis subject (b) establish an optimal rate of infusion of a separated blood component to said subject (c) effect the operation of said one or more pumps at said optimal infusion rate.

### Brief Description of the Drawings

FIG. 1 is a perspective view of an exemplary durable blood processing system that may be used in accordance with the present disclosure;
FIG. 2 is a perspective view of a disposable set usable in association with the durable fluid processing system of FIG. 1; and
FIG. 3 is a side view of the embodiment of the disposable set shown in FIG. 2 mounted on the embodiment of the durable system shown in FIG 1.

### Detailed Description of the Embodiments

The embodiments disclosed herein are for the purpose of providing a description of the present subject matter. It will be understood that the subject matter may be embodied in various other forms and combinations not shown in detail. Therefore, specific designs and features disclosed herein are not to be interpreted as limiting the subject matter as defined in the accompanying claims.

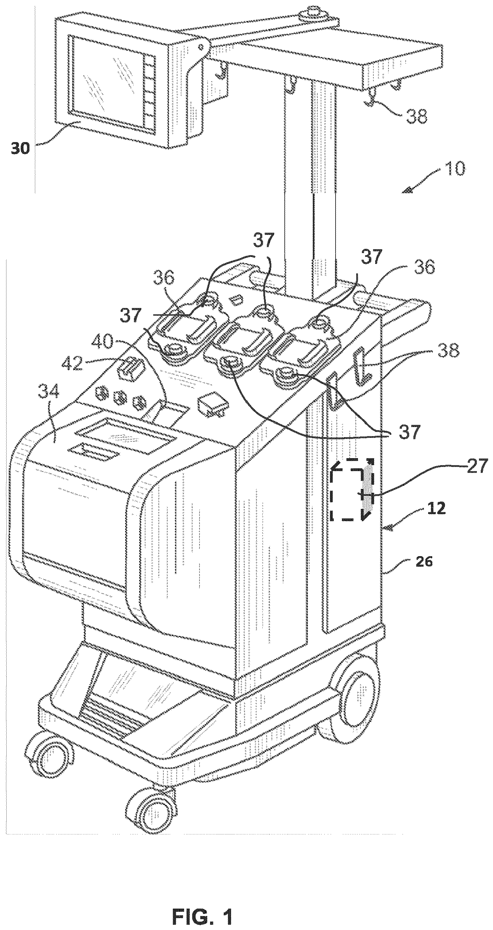

FIG. 1 shows an apheresis system 10 in accordance with the present disclosure. The system includes many of the features present in an apheresis system currently marketed as the AMICUS^{®} Separator by Fenwal, Inc. of Lake Zurich, Illinois, a subsidiary of Fresenius-Kabi of Bad Homburg, Germany, and described, for example in U.S Patent No. US 9,176,047, the contents of which are incorporated herein by reference in their entirety.

The blood processing system 10 can be used for processing various fluids, but is particularly well suited for processing whole blood, blood components, or other suspensions of biological cellular materials. The system 10 includes a durable, reusable blood separation device 12 for separating a fluid into its constituent parts. A more detailed description of the blood separation device 12 and the other elements of the system 10 can be found in U.S. Patent No. 5,996,634, which is incorporated herein by reference. While FIG. 1 illustrates a centrifugal blood processing system, it should be understood that the illustrated system is merely one example, and other blood processing systems (including filter- or membrane-based blood separators and other centrifugation or other separation systems) may be used according to the present disclosure. Systems using a spinning membrane for separating blood into its components are described in U.S. Patent Nos. 11,383,013; 10,946,131; 11,097,042; 11,285,251 and 11,369,724, the contents of all of which are incorporated herein by reference.

When used for processing blood, a blood component, or any other body fluid, the systems, devices, and methods according to the present disclosure are typically used with living subjects (as opposed to previously collected blood or biological fluid wherein a container of blood or biological fluid serves as the source). For example, the fluid source may be a living human or animal whose bodily fluid is directly drawn into the device for processing.

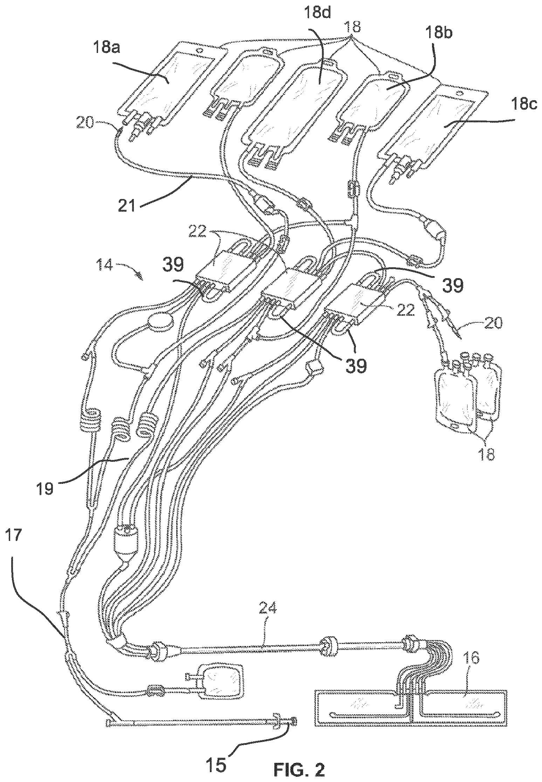

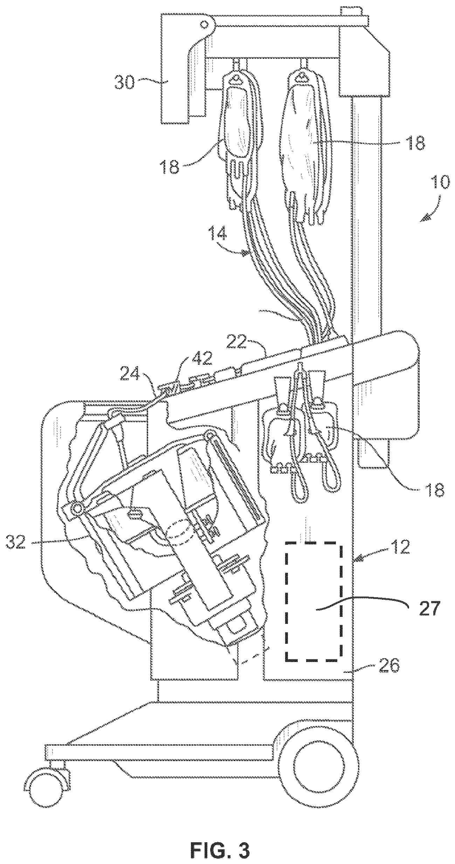

The durable blood processing device 12 is used in combination with a disposable processing set 14, an example of which is shown in FIG. 2. FIG. 3 shows the disposable set 14 mounted on the durable device 12. The disposable set 14 is a preferably single use, disposable item loaded on the system 10 at the time of use. After a fluid processing procedure has been completed, the operator preferably removes the disposable set 14 from the system 10 and discards it. It should be understood that the illustrated disposable set 14 of FIG. 2 is merely one example of a fluid circuit. The illustrated disposable set 14 is suitable for use with a system 10 as shown in FIG. 1, but those of ordinary skill in the art will appreciate that differently configured disposable sets are appropriate for use with different blood processing systems.

The disposable set 14 includes a processing chamber 16 (FIG. 2). In use, the device 12 rotates the processing chamber 16 to centrifugally separate blood components. Whole blood is conveyed to the processing chamber 16, and separated blood components are conveyed from the processing chamber 16 through a plurality of flexible tubes that form part of a fluid circuit. The fluid circuit further includes a plurality of containers 18 that may be supported by elevated hangers located over the device 12 (see FIG. 3) and that dispense and receive liquids during processing. At least some of the containers 18 are separate from the disposable set 14 and connected thereto by connectors 20. The nature of the connectors 20 may vary without departing from the scope of the present disclosure. For example, the connectors 20 may be cannulas configured to puncture the containers 18 to establish fluid communication between the fluid circuit and the containers 18. Other connectors and means for connecting separate containers to a disposable set 14, such as sterile connection devices, are known to those of ordinary skill in the art and may be employed without departing from the scope of the present disclosure. Preferably, the disposable set 14 is a pre-assembled closed system, with the connectors 20 allowing for sterile connection of the containers 18 to the fluid circuit.

Fluid flow through the disposable set 14 may be controlled in a variety of ways. In one embodiment, fluid flow is controlled via cassettes 22 with pre-formed fluid passageways, which may be selectively opened and closed pneumatically, hydraulically, or by movable actuators. The number of cassettes may vary, but in the illustrated embodiment, there are three cassettes 22, which operate in association with valve and pump stations on the centrifuge device 12 to direct liquid flow among multiple liquid sources and destinations during a blood processing procedure. Tubes connected to the processing chamber 16 lead to a flexible umbilicus 24, with additional tubes at the other end of the umbilicus 24 fluidly connecting the processing chamber 16 (via the umbilicus 24) to the remainder of the disposable set 14, including the containers 18 and the cassettes 22.

As illustrated, the device 12 includes a wheeled cabinet 26 that can be easily rolled from place to place. A user-actuated processing controller 27 is provided which enables the operator to control various aspects of the blood processing procedure. A centrifuge rotor assembly 32 is provided behind a fold-open door 34 (FIG.1) that can be pulled open at the front of the cabinet 26. A plurality of valve and pump stations 36 (FIG. 1) are provided on the top face of the cabinet for receiving and controlling the various cassettes 22. As shown in FIG. 1, each pump station 36 includes a pair of (peristaltic) pump rotors 37 configured to receive mating tubing loops 39 of cassettes 22. A plurality of hooks or hangers 38 are provided on the cabinet 26 for suspending the various containers 18.

In use, the fold open door 34 is opened and the processing chamber 16 of the disposable set 14 is mounted in the centrifuge rotor assembly 32. The umbilicus 24 is threaded through the centrifuge rotor assembly 32 and out through an opening 40 in the upper panel of the cabinet 26 (FIG. 3). The cassettes 22 are snapped into respective ones of the valve and pump stations 36 and the containers 18 are hung from the appropriate hangers 38 (FIG. 3). After appropriate connections are made to the patient or donor using known intravenous techniques, the operator enters appropriate commands on the processing controller 27 to begin the desired processing procedure.

The durable hardware device 12 includes a controller, such as the controller 27 generally shown in FIGS. 1 and 3. Although shown in the cabinet 26 of the durable hardware device 12, the controller may be incorporated in a different part of the device 12. The controller 27 may, according to the embodiments described herein, include a programmable microprocessor, which may be programmed to operate the blood processing device 12 and system 10 according to a process.

According to other embodiments, the controller may include one or more electrical circuits designed to carry out the actions described herein. In addition, the controller may include one or more memories. The instructions by which the microprocessor is programmed may be stored on the memory associated with the microprocessor, which memory/memories may include one or more tangible non-transitory computer readable memories, having computer executable instructions stored thereon, which when executed by the microprocessor, may cause the microprocessors to carry out one or more actions as described below.

The controller 27 may be coupled to one or more of the structures of the blood processing device 12 and also to the structures of the disposable set 14, for example to receive information (e.g., in the form of signals) from these structures or to provide commands (e.g., in the form of signals) to these structures to control the operation of the structures. The controller may be coupled to the sensors, valves, and pumps to provide commands to those devices to control their operation. It may also be possible that the controller receives information from and provides commands to a given structure, such as one of the structures already mentioned. The controller 27 may be directly electrically connected to these structures to be coupled to them, or the controller may be directly connected to other intermediate equipment that is directly connected to these structures to be coupled to them.

The controller 27 is configured and/or programmed to execute at least one blood processing procedure but, more advantageously, is configured and/or programmed to execute multiple types of blood processing procedures which may include a separating portion and a return portion.

More particularly, in carrying out any blood processing procedure, the controller is configured and/or programmed to control the flow of fluid and amount of a fluid from one component to another. This may include instructing the valves to open and close at specific points during a procedure or initiating transfer of a fluid from one container to another. Hence, while it may be described herein that a particular component of the blood processing system performs a particular function, it should be understood that that component is being controlled by the controller to initiate and/or perform that function.

A user interface screen 30 (e.g., a touchscreen) may be associated with the blood processing device 12 (as in FIGS. 1 and 3). The user interface screen 30 may allow an operator to interact with the system controller 27 (e.g., a microprocessor) of the device 12 to provide instructions to the controller (e.g., to carry out a particular procedure), as well as provide information to the controller to be used during a procedure, including entry of apheresis subject characteristics and other parameters. The user interface screen 30 may also serve as a display to provide the operator with instructions (e.g., to connect or disconnect the blood source from the disposable set 14) and information (e.g., alerting the operator to a blockage in a fluid flow conduit of the disposable set 14) and provide a procedure status.

In one example of an apheresis procedure, whole blood or a fluid containing one or more blood components is drawn into the disposable set 14 by a phlebotomy needle 15 or other access device (FIG. 2). To reduce clotting, citrate anticoagulant is added to the fluid from one of the containers 18, e.g., 18a via line 21. The anticoagulated blood flows into the processing chamber 16 via draw/inlet line 17, where it is separated into at least two components based on their relative densities (e.g., a relatively high-density component, such as red blood cells, and a relatively low-density component, such as platelet-rich plasma). One of the components (the high-density component in one embodiment) may be pumped out of the processing chamber 16 and into one of the containers 18, e.g., 18b, which serves as a storage container. The storage container may be provided with an amount of storage fluid or, alternatively, storage fluid may be added to the storage container from one of the other containers 18, e.g., 18c. The other component (the low-density component in one embodiment) may be pumped out of the processing chamber 16 and returned to the blood source via return line 19. As noted above, the component being returned to the apheresis subject through return line 19 will include some amount of citrate anticoagulant (having been derived from anticoagulated whole blood). Replacement fluid, such as saline (from container 18d), may be added to the fluid that is returned to the blood source to compensate for the blood volume deficit caused by storage of the selected blood component(s) in the storage container after separation.

Turning now to the method of performing an apheresis procedure in accordance with the present a disclosure, the risk of a citrate reaction may be predicted and at least partially mitigated by using a donor characteristic such as body water when determining a flow rate for the components being returned to the apheresis subject. Depending on the subject and his/her volume or percentage of body water, such flow rate can be safely increased, thereby potentially reducing the duration of the apheresis procedure.

"Body water" may include the subject's total body water, plasma volume, extracellular fluid volume or ECFV. Body water can be calculated from an experimentally derived equation or physiologic measurement of the donor or patient or a combination thereof. In one embodiment, body water volume may be based on one or more of the subject's age, height, weight, and gender, pursuant to Watson's Formula for men and women. Using Watson's Formula, one can arrive at a volume (liters) of total body water (TBW) for males as follows: 2.447 - (0.09145 x age) + (0.1074 x height in centimeters) + (0.3362 x weight in kilograms) = total body water (TBW) in liters. Similarly, using Watson's Formula, one can arrive at a volume (liters) of total body water for females: -2.097 + (0.1069 x height in centimeters) + (0.2466 x weight in kilograms) = total body water (TBW) in liters. See Watson et al., "Total body water volumes for adult males and females estimated from simple anthropometric measurements," The American Journal of Clinical Nutrition, Volume 33, January, 1980, pp.27-39, the contents of which is incorporated by reference. Other methods for calculating Total Body Water (TBW) are described in Castro et al., "Equation for Estimating PreDialysis Total Body Water in Patients under Chronic Hemodialysis," American Journal of Biomedical Science & Research, 9(5) (2020). (https://biomedgrid.com/pdf/AJBSR.MS.ID.001436.pdf).

As described above, "body water" may also refer to a subject's Total Plasma Volume (TPV) which can be arrived at by using one or more of subject's weight, height, gender, and hematocrit. Methods for determining the TPV of a subject are described in U.S. Patent Nos. 11,383,013; 10,946,131; 11,097,042; 11,285,251 and 11,369,724, the contents of all of which are incorporated herein by reference. As set forth in the patents identified above, TPV may be estimated with reference to the weight or weight range as set forth in established FDA nomograms or other guidelines.

"Body water" may also be the extracellular fluid volume or ECFV of the donor or patient, as described in International Application Publication No. WO 2023/027780, which is herein incorporated by reference. Extracellular fluid may comprise interstitial fluid, blood plasma, lymph, and transcellular fluids (e.g., cerebrospinal fluid, fluid in the gastrointestinal tract, etc.). Extracellular fluid amounts can be estimated or calculated in any number of different ways. In one example, extracellular fluid volume (ECFV) of the donor may be based on the weight (BM) and height (H) of the donor such that: ECFV (L) = 0.02154 × BM^{0.6469} (kg) × H^{0.7236} (cm) where ECFV is expressed in liters, BM is expressed in kilograms and H is expressed in centimeters. Bird et al., "Indexing Glomerular Filtration Rate to Suit Children", J. Nucl. Med 2003; 44:1037-1043 (Bird 2003). An alternative expression in SI units is: ECFV (L) = 0.6032 × BM^{0.6469} (kg) × H^{0.7236} (m) where H is expressed in meters.

In another example, extracellular fluid volume (ECFV) of the donor may be based on the weight (W) and height (H) of the donor such that: ECFV (L) = aW^{b}H^{c} where a=0.0399, b=0.6065, and c=0.6217 for females and a=0.0755, b=0.6185 and c=0.4982 for males, and where weight (W) is expressed in kilograms and height (H) is expressed in centimeters. Bird, N. J. and Peters, A.M., "New gender-specific formulae for estimating extracellular fluid volume from height and weight in adults", Nuclear Medicine Communications, 42:58-62 (2021) (Bird 2021).

In another example, extracellular fluid volume (ECFV) of the donor may be based on the weight (BM) and height (H) of the donor such that: ECFV (L) = sqrt(BM (kg)) x H (m) where ECFV is expressed in liters, BM is expressed in kilograms and H is expressed in meters. Abraham et al., "Extracellular Volume and Glomerular Filtration Rate in Children with Chronic Kidney Disease", Clin. J. Am. Soc. Nephrol., 2011 Apr; 6(4): 741-747.

In another example, extracellular fluid volume of the donor may be based on the weight (BM) and height (H) with a coefficient, such that: ECFV (L) = 0.96 × BM^{0.51} × H^{0.96}.

In another example, extracellular fluid amount can be calculated as extracellular fluid weight (ECFW) based on the weight (BM) of the donor such that: ECFW (kg) = 0.135 x BM (kg) + 7.35 kg where ECFW and BM are expressed in kilograms.

In still another example, extracellular fluid volume (ECFV) of the donor may be based on the weight (BM) and height (H) of the donor such that: ECFV (L) = 0.0682 × BM^{0.400} (kg) × H^{0.633} (cm), where ECFV is expressed in liters, BM is expressed in kilograms and H is expressed in centimeters. Friis-Hansen, "Body Water Compartments in Children: Changes During Growth and Related Changes in Body Composition", Pediatrics; vol. 28 no. 2, Aug. 1961.

In yet another example from Friis-Hansen, extracellular fluid volume (ECFV) of the donor may be based on weight alone (BM) such that: ECFV (L) = 0.583 x BM^{0.678} where ECFV is expressed in liters and BM is expressed in kilograms.

In other examples, extracellular fluid amount may be calculated by first calculating total body fluid (TBF) amount using any known algorithm, calculating an intracellular fluid (ICF) amount, and subtracting ICF from TBF. Other calculations and/or estimations of extracellular body fluid are also contemplated.

Using the user interface screen 30, the operator may select which method for estimating body water to use. The system may be pre-programmed with the different ways of calculating described above for selection by the operator. Once selected, the system under the direction of the controller 27 may prompt the operator to enter any required physiological characteristics of the donor or patient, such as height, weight, gender, age, etc. for the selected body water calculation methodology (e.g., total body water, total plasma volume, ECFV etc.). The controller will then calculate an estimated body water volume. Based on the calculated or estimated body water volume and the estimated amount of citrate in the component(s) being returned (discussed below), the system will then establish a suitable and safe return flow rate for the apheresis procedure as described below.

The amount of citrate in the component(s) being returned will depend on several factors including the composition of the citrate solution that is added to the whole blood during the whole blood draw. Anticoagulants commonly used in apheresis procedures typically include trisodium citrate, citric acid and dextrose and include Acid Citrate Dextrose solution A (ACD-A) and Acid Citrate Dextrose Solution B (ACD-B). ACD-B differs from ACD-A in that it contains lower concentrations of citrate, citric acid and dextrose. Other citrate-containing anticoagulants are also known such as Citrate Phosphate Dextrose (CPD) which includes citric acid, dextrose and sodium phosphate buffer, although in apheresis procedures, ACD-A or ACD-B may be preferred.

In addition to the composition of the citrate solution (anticoagulant), the amount of citrate being returned to the donor or patient will also depend on the amount of the citrate solution combined with the whole blood that is withdrawn from the donor or patient. Typically, the relative amounts of anticoagulant and whole blood are established as a ratio (by volume) of the whole blood to the volume of the citrate solution may be used to determine the amount the citrate in the components being returned. Typical whole blood to anticoagulant ratios (WB:AC ratios) for apheresis procedures may range from 10:1 to 16:1, although other ratios may also be used. The above-mentioned factors and the flow rate in the return line are used to determine the citrate infusion rate.

Other parameters which may be taken into account (and programmed into the control system of the apheresis device) when arriving at a citrate infusion rate and/or return rate of the blood components is the donor's hematocrit (Hct), which may be indicative of a donor's ability to metabolize citrate. Hematocrit may be determined at the beginning of the procedure and/or during the procedure (inasmuch as donor Hct may change during the course of the procedure). Hematocrit may be determined optically using sensors arranged in proximity to selected flow paths of the apheresis system and/or at the interface of blood components as they are being separated in the separation chamber 16 (FIG. 3) of the device.

Still other information that may be considered in determining suitable citrate infusion rates (and also programmed into the control system of the device) include historical data for the given donor, such as the timing and severity of citrate reactions during earlier donations.

Control of the return flow rate may be achieved in one or more ways. In one embodiment, where the apheresis subject has a "high" body water volume or percentage, the system will establish a "higher-than-normal" return flow rate, and the controller will actuate the return pump 36 and maintain the pump speed to achieve the established flow rate. The controller may include a pre-programmed body water threshold whereby an apheresis subject with a body water volume or percentage above such threshold will result in the controller establishing a higher-than-normal return flow rate and pumps speed. Subjects falling below the body water threshold will have components returned at a slower or default flow rate and reduced pump speed.

Return flow rates may be in the range of 60-120 mL/min, although other ranges may be used and/or the range adjusted based on other factors. For example, the return flow rate may also depend on (and even to a large degree) on the caliber of the vein in which that the return line needle is inserted.

Alternatively, rather than having pre-set thresholds or "cut-offs," the maximum flow rate may be determined by the controller based on the total body water of the patient/donor as well as the citrate content in the return fluid (i.e., setting a maximum citrate infusion rate per mL of total body water). That maximum citrate infusion rate may be, by way of example only, 111 mL/min, 110 mL/min, 109 mL/min etc, but in all cases, below a strict maximum pre-programmed into the controller. The pre-programmed maximum provides a safety setting to ensure that the donor/patient's vein does not burst due to high infusion pressure.

In a further embodiment, the maximum infusion rate (maximum citrate infusion rate) may vary during the procedure to account for the fact that citrate may gradually accumulate within the donor/patient's body as the procedure progresses. Accordingly, the maximum infusion rate may be higher at the beginning of the apheresis procedure and then gradually decrease near the end of the procedure. The change in infusion rate over time may be adjustable by the operator/user since the optimal setting may vary from donor to donor as well as depend on practices at the center (such as whether they administer calcium supplements at the start of the procedure).

The description provided above is intended for illustrative purposes only and is not intended to limit the scope of the invention to any specific method, system, or apparatus, or device described herein except as may be explicitly delineated above.

## Claims

1. A method for programming a controller to implement a biological fluid processing procedure for controlling a risk of a citrate reaction in an apheresis subject comprising:
a) determining a physical parameter of an apheresis subject;
b) determining an amount of body water in the apheresis subject based on said physical parameter;
c) carrying out an apheresis procedure on said apheresis subject, said procedure comprising infusing a separated citrate-anticoagulated blood component to said subject; and
d) establishing a rate of infusion of said separated citrate-anticoagulated blood component to said subject based at least, in part, on said amount of body water in said apheresis subject.

2. The method of Claim 1 wherein physical parameter of said apheresis subject is selected from one or more of body weight, body mass index, plasma volume, extracorporeal fluid volume.

3. The method of any one of Claims 1 and 2 further comprising adjusting said rate of infusion based on the volume of body water in said apheresis subject.

4. The method of any one of Claims 1 through 3 comprising establishing fluid communication between the vascular system of the apheresis subject and a cell separation device.

5. The method of any one of Claims 1 through 4 wherein said amount of body water in said apheresis subject is based on one or more of the total body water, total plasma volume and extracellular fluid volume.

6. The method of any one of Claims 1 through 4 wherein the amount of body water is based on one or more of the age, height, weight, gender of the apheresis subject.

7. A system for carrying out an apheresis procedure comprising:
a. a durable, reusable hardware device including a cell separator drive unit, one or more pumps and a controller;
b. a disposable fluid circuit configured for mounting onto said hardware device, said fluid circuit including a separation chamber, a venous access device for establishing flow communication between an apheresis subject and said separation chamber, said circuit including a line defining a flow path for introducing blood from said apheresis subject to said separation chamber and a line for infusing a separated blood component from said chamber to said apheresis subject; and
c. wherein said controller is configured to (a) determine the body water volume of an apheresis subject (b) establish an optimal rate of infusion of a separated blood component to said subject (c) effect the operation of said one or more pumps at said optimal infusion rate.

8. The system of Claim 7 wherein said fluid circuit is configured for attachment to a source of a citrate anticoagulant and further includes a line defining a flow path for combining anticoagulant with blood withdrawn from the apheresis subject.

9. The system of Claim 8 wherein the volume of citrate anticoagulant combined with said blood is based on the amount of blood withdrawn from the apheresis subject.

10. The system of Claim 9 wherein said anticoagulant source comprises a container of citrate anticoagulant associated with a weight scale.

11. The system of any one of Claims 7 through 10 wherein said separation chamber includes a fluid outlet through which a separated blood component flows, said system further comprising a return line defining a flow path between said outlet and said apheresis subject.

12. The system of Claim 11 wherein said controller is further configured to determine the volume of citrate in said separated blood component, in particular wherein said return line is associated with a return pump and said controller is further configured to effect operation of said return pump and infuse said separated blood component at said optimal infusion rate.

13. The system of any one of Claims 7 through 12 wherein said controller is configured to determine said amount of body water based on one or more of an apheresis subject's age, weight, height, and gender,.

14. The system of Claim 13 wherein said controller is configured to determine said amount of body water by determining at least one of the total plasma volume and extracellular fluid volume of the apheresis subject.

15. The system of any one of Claims 7 through 14 wherein said separation chamber comprises a centrifuge or a spinning membrane.
